Europäisches Patentamt

**European Patent Office**

Office européen des brevets

(11) Publication number: **0 077 413**

**A1**

(12) **EUROPEAN PATENT APPLICATION**

(21) Application number: **81108571.1**

(22) Date of filing: **20.10.81**

(51) Int. Cl.³: **G 01 F 1/56**
**A 61 B 5/00**

(43) Date of publication of application:
**27.04.83 Bulletin 83 17**

(84) Designated Contracting States:
**DE FR IT**

(71) Applicant: **Memorial Hospital for Cancer and Allied Diseases**
**1275 York Avenue**
**New York, N.Y. 10021(US)**

(72) Inventor: **Rottenberg, David**
**1161 York Avenue**
**New York, N.Y. 10021(US)**

(72) Inventor: **Miodownik, Saul**
**1275 York Avenue**
**New York, N.Y. 10021(US)**

(72) Inventor: **Epifanio II, W.E.**
**139 Emery Drive East**
**Stamford, Conn. 06902(US)**

(74) Representative: **Zapfe, Hans, Dipl.-Ing.**
**Seestrasse 2**
**D-6054 Rodgau-3(DE)**

(54) **Method and apparatus for electrically measuring fluid flow rates.**

(57) A system for measuring the flow rate of electrolytic fluids includes a flow cell (10) with a cylindrical channel for the fluid flow and a pair of spaced apart electrodes conformed to the cylindrical surface of the flow channel. The electrodes are electrically conductive and inert to the fluid and are in contact with the fluid flow for a desired range of flow measurement. A monopolar pulse train is applied across the electrodes to effect a cell impedance which is inversely proportional to the rate of flow. A control signal is produced in response to the potential across the electrodes and this signal is applied to the pulse train generator varying the pulse train current to effect an increase in the current in response to an increase in flow rate whereby the cell impedance is stabilized for every flow rate and the electrode potential is representative of the flow rate.

FIG. 4.

EP 0 077 413 A1

Sloan-Kettering Institute for Cancer Research
1275 York Avenue
New York, N.Y. 10021
United States of America

---

Method and Apparatus for Electrically Measuring
Fluid Flow Rates

---

BACKGROUND OF THE INVENTION

This invention relates to a method and apparatus for flow measurement, and more particularly to the measurement of very slow flow rates of electrolytic body fluids.

Numerous devices have been proposed and developed for measuring the rate of flow of fluid to various conduits. However, flowmeters which are commonly used for measuring relatively low flow rates either fail to accurately measure the low flow rates and/or simply take too long to make the measurement.

- 2 -

One well-known class of flowmeters is called the head meter which functions by measuring the pressure differential across a suitable restriction to flow into a pipe conducing the flow to be metered. The pressure differential may be created by an orifice plate, a venturi restriction, a capillary tube or other form of primary. The pressure differential developed across the primary of a head meter is measured by pressure responsive secondaries having deflectable metal diaphragms. Very low flow rates suffer inaccuracies due to the history in the deflection vs. differential pressure characteristics inherent in metal diaphragms. Morever, response time may extend to minutes or hours when the flow rate is so low that it is only capable of satisfying the volume displacement of the diaphragm in minutes or hours. Also, capillaries foul easily or if made so large to prevent fouling may take hours before fluid entering one end emerges from the other.

Another type of flowmeter is the area type where a variable orifice and a substantially constant pressure drop rather than a fixed orifice and a varying pressure drop as a function of flow rate is measured. In the area meter, flow rate is reflected by the changing area of the annular opening through which the liquid must pass. In one such standard variable area flowmeter of the type disclosed by Dettmer U.S. Patent No. 3,712,134, a vertical tube through which the fluid is conducted in the upward direction is provided with a tapered bore affording a variable cross-sectional area. A weighted float is disposed in the bore and is caused to assume a vertical position representing a condition of equilibrium between the downward gravitational force on the float through the annular orifice which surrounds it. The position of equilibrium is therefore a function of the flow rate, and the greater the flow rate, the higher the vertical position of the float. The variable area flowmeter, however, cannot be used for extremely low flow rates because of the friction and other forces which load the float causing large errors in drag which distort the relationship between the flow rate and the float position and thereby give rise to inaccurate readings.

In patent 3,450,984 a pair of electrodes are inserted in a fluid and a polarizing current capable of electrolyzing the fluid is passed between the electrodes. Electrolysis causes an accumulation of gas bubbles in the current path of the electrodes and the gas bubbles are displaced at a rate depending upon the electrolyte flow rate resulting in a change in resistance between the electrodes which is an indication of electrolytic flow rate. The problem with this technique is that the response time is very, very long and reproducibility is difficult to achieve. The current between the electrodes must stabilize before an accurate measurement can be made.

The above mentioned prior art flow meters and methods of measuring flow have a further disadvantage of not being capable of adequately measuring the flow of cerebrospinal fluid in a ventriculoatrial shunt and/or a ventriculoperitoneal and lumboperitoneal shunt, for which the need for such measurements has increased due to the increase usage thereof. Cerebrospinal fluid shunt flow is particularly difficult to measure due to the intermittancy thereof and the various pressure gradients exerted thereon due to changes in physiological conditions. Moreover, since the shunt tubing itself is generally implanted below the skin of the patient, it is highly desirable that any flow cell associated therewith also be implantable. Lastly, due to the fact that the flow measurement is in vivo and carried out during the use of the shunt on the patient, it is important that the flowmeter itself does not in any way obstruct or change the dynamics of the flow of the spinal fluid during use.

## SUMMARY OF THE INVENTION

Accordingly, it is an object of the present invention to provide a new and improved method and apparatus for measuring the flow rates of a fluid which is simple, reliable and has a fast response time.

Another object of this invention is to provide a new and novel method and apparatus for measuring slow flow rates while minimizing gross disturbances in the flow rates being measured.

Still another object of this invention is to provide a new and improved method and apparatus for measuring flow rates which are extremely slow and which are capable of measuring the flow rates of body fluids such as cerebrospinal fluid flow rates.

A further object of the invention is to provide a new and improved flow cell which is particularly useful for the measurement of cerebrospinal fluid in a shunt, that is the flow cell does not disturb the flow rate being measured and is capable of being implanted with the shunt in a patient.

These and other objects of the present invention are achieved in accordance with the present invention by a flow cell including means forming a cylindrical flow channel receptive of the fluid flow to be measured and a pair of spaced apart electrodes which are configured to conform to the cylindrical surface of the flow channel and to be flushed therewith. The electrodes comprise electrically conductive material which is inert to the fluid of the flow to be measured and the channel is configured to effect contact of the electrodes with the fluid flow for a desired range of flow rate measurement.

In order for the flow cell to be implantable in the body, in a particularly advantageous preferred embodiment, the diameter of the flow channel is substantially equal to the diameter of the shunt tube with which it is used and the means forming the flow channel has a cylindrical outer surface, the diameter of which is substantially equal to that of the outer diameter of the shunt tube with which it is being used.

The method and device for measuring the flow rate of an electrolytic fluid through the aforesaid flow cell is based upon the belief that if a pulse is applied across the flow cell electrodes having a selected amplitude, period and pulse width, electrolysis of the fluid will take place with the result that the cell impedance across the electrodes will be inversely proportional to the rate of flow. It is believed that this relationship is caused by the fact that the flow strips bubbles from the surface of the electrodes and thus decreasing the resistance with an increase in flow rate. Also in accordance with the invention, it has been observed that as one increases the current applied across the electrodes in a given unit of time either by increasing the amplitude or preferably by increasing the duty cycle of the pulse applied across the electrodes, the impedance will increase.

Thus the present invention is based upon the concept that the resistance of the flow cell is directly proportional to the current applied across the electrodes in a given unit of time and inversely proportional to the rate of flow during that unit of time. Given this fact, the resistance of the flow cell can be stabilized by a negative feedback control system which varies the current applied across the electrodes in a given unit of time in response to a change in the flow rate whereupon the resulting potential across the electrodes is a direct function of the actual flow rate and is therefore representative thereof to provide a measurement value for the flow rate.

Accordingly, means for applying a monopolar pulse train across the flow cell electrodes is provided, the means being controllable to vary the current applied across the electrodes in a given unit of time in response to a control signal which preferably alters the duty cycle of the pulse train to effect an increase or decrease in current. The control signal is produced by sensing the potential across the electrodes for each given unit of time and applying the control signal as a negative feedback input to the means for applying the pulse train to effect an increase in current in response to an increase in the flow rate or a decreasing current in response to a decreasing flow rate so as to stabilize the cell impedance for each flow rate.

Since the pulse applying means and means for producing the control signal are capable of being produced by integrated circuits, the present invention makes it possible to either implant only the flow cell with the terminals of the electrodes accessible for applying the pulse train, or, implanting the entire flow cell and circuitry in the patent with or without a power source therefor, so that only the output of the circuit and/or the power for actuating same need be accessed or supplied.

- 8 -

Advantageously, a near perfect relationship between the systems output voltages and flow rates is provided. The system is also flexible enough to provide a range of flow rates which can be varied merely by altering the current density through the flow cell. Flow rates data may be obtained spanning over two orders of magnitude for a given set of circuit parameters.

BRIEF DESCRIPTION OF THE DRAWINGS

The invention, together with further aspects, objects and advantageous thereof, will be better understood from the following description taken in connection with the accompanying drawings.

FIG. 1 is an isometric view of a flow cell which may be employed in the present invention.

FIG. 2 is a partial enlarged view of a pair of spaced electrodes which may be utilized in the flow cell shown in FIG. 1.

FIG. 3 is a block diagram of the system according to the invention.

FIG. 4 is an electrical schematic diagram of an illustrative embodiment of a circuit for the system of FIG. 3.

DESCRIPTION OF THE PREFERRED EMBODIMENTS

In accordance with the method and apparatus for measuring slow flow rates in electrolytic fluids as embodied in the present invention, the system includes two principle parts, one of which is the electrolytic flow cell through which the solution to be measured passes and the other is the electronic circuitry to which the electrolytic flow cell is coupled for making the desired measurement.

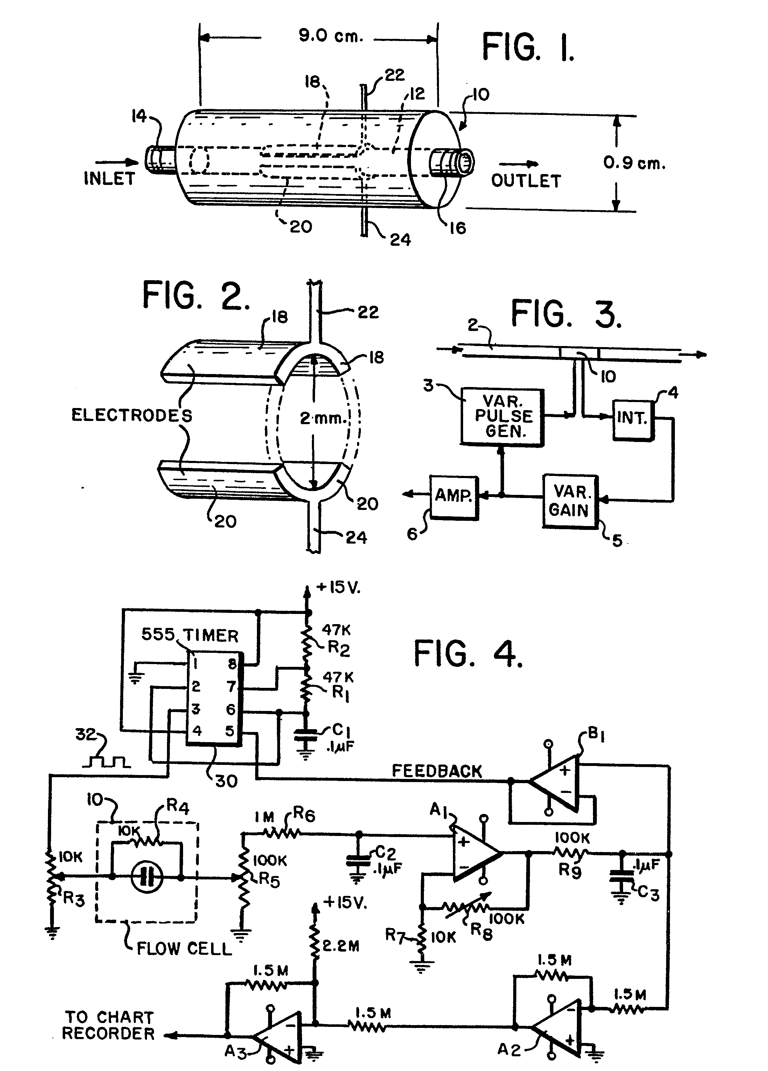

Referring now to FIG. 1 and 2 which illustrate one type of flow cell which may be utilized in the present invention, the flow cell referred to generally with the reference character 10 is generally cylindrically shaped having a central channel 12 therein with an inlet port 14 on one end thereof and an outlet port 16 on the other end thereof. The fluid flow through the flow cell 10 is indicated by the arrows on the drawing in FIG. 1.

The flow cell 10 has a pair of electrodes 18 and 20 positioned parallel to each other and parallel to the external cylindrical walls of the cell. The electrodes 18 and 20 partially surround and are recessed in the channel 12 of the flow cell 10 so as to maintain contact with the fluid moving through the cell while minimizing any gross disturbances in the flow of the fluid which is being measured. Electrodes 18 and 20 have electrical contact leads 22 and 24, respectively connected thereto which couple the flow cell 10 to the electronic portion of the circuitry which will be described hereinafter. The electrodes represent the only portion of the system in actual contact with the moving solution. In order to assure the stability and reproducibility of the flow cell 10 electronic characteristics, it is important that no reactions occur at the electrode surfaces. Accordingly, gold, platinum or any other inert electrode material is preferred. The type of electrode material used will depend upon the particular application and the type of solution whose flow characteristics are being measured. All the parameters of the electrode geometry have an effect on circuit operation and accordingly the particular size and spacing will depend upon the type of measurement being made, the flow characteristics of the fluid being measured as well as other parameters for a particular application.

FIG. 3 shows a block diagram of the system for use with the flow cell 10 for carrying out the flow measurement according to the present invention.

As shown in Fig. 3, the flow cell 10 is connected in series in a flow line 2 such as a tube or the like. The flow measuring circuitry which is attached to the electrodes of flow cell 10 comprise circuit elements 3-6. Variable pulse generator 3 applies a pulse train across the flow cell electrodes, the pulse train having a selected amplitude, period and duty cycle and the variable pulse generator 3 is capable of varying the amplitude, pulse width or period of the pulse train in order to vary the amount of current applied to the flow cell in a given unit of time. Preferably, the given unit of time is defined by a fixed period for the pulses having a fixed amplitude so that the current is varied only by the pulse width per period.

The principle of the invention is based upon the fact that when a monopolar pulse is applied across the electrodes, bubbles will be formed at the electrodes in some proportion to the current applied during each period. When bubbles are formed on the electrodes, this reduces the surface area of the electrodes through which the current can pass and as a result, the impedance between the electrodes will increase, thereby reducing the current and the amount of bubbles formed. At the same time, the fluid flow itself strips away bubbles at the electrodes and the number of bubbles which are stripped away is in some way a function of the flow rate. Thus, the impedance of the flow cell is directly proportional to the current applied thereacross for each pulse period and inversely proportional to the flow rate.

At the output of the flow cell electrodes, an integrator 4 is provided which integrates the pulsed current output from the electrodes so as to obtain some dc or constant current value which will change for changes in flow rate or changes in current applied across the electrodes per period. This output from the integrator 4 is fed back to the pulse generator 3 through a variable gain element so that the amount of current per period is changed by the variable pulse generator 3 when the flow rate changes.

- 12 -

As can be clearly seen, since the impedence of the flow cell electrodes is directly proprotional to the current per period passing therethrough and inversely proportional to the flow rate, if the flow rate should change, the current can be likewise changed to offset the change in flow rate so as to stabilize the flow cell impedance. This stabilization can be carried out relatively quickly by the negative feedback loop shown in Fig. 3.

Thus, given that there is a constant flow passing through the tube 2 for which the variable pulse generator 3 has generated a given pulse width per period which has stabilized the flow cell impedance, if the flow rate increases, the impedance of the flow cell electrodes will decrease thus increasing the steady state or constant value from integrator 4. This increase in the output of integrator 4 effects an increase in the pulse width of the pulse train being applied to the flow cell whereby the current applied to the pulse flow cell increases and thereby the resistance thereof will increase due to increase bubble formation. Thus a constant or stabilized flow cell impedance will be achieved for this new flow rate. If the flow rate should decrease, the electrode impedance will increase thereby decreasing the output of the integrator 4 which will then decrease the width of the pulses generated by the pulse generator 3 to stabilize the impedance again.

It can be therefore recognized that the feedback control voltage applied from gain element 5 to variable pulse generator 3 is directly representative of the flow rate since it increases as the flow rate increases and decreases as the flow rate decreases and that there is a particular value of this control voltage for each flow rate. Thus passing the control voltage through an amplifier 6 produces a signal which corresponds to the flow rate measurement and which can be displayed on a chart recorder or some digital display to indicate the actual flow rate.

One way of achieving the required stabilization is illustrated in one form in the electronic circuitry illustrated in FIG. 4. A monopolar pulse generator 30, illustrated in the form of a 555 integrated circuit timer chip, generates pulses 32 of a 14 volt magnitude at a frequency of approximately 100 Hz. The frequency and duty cycle of the wave form 32 are controlled by the combined values R1, R2 and C1 connected to the circuit 30 as shown as well as the voltage applied to pulse width modulation control input pin 5 which will be described hereinafter. Since the component values of R1, R2 and C1 are fixed, the pulse generator 30 relies on changes in modulation input voltage which is the signal applied to pin 5 in order to affect changes in output duty cycle and/or frequency. Accordingly, the pulse generator 30 serves as a variable pulse width pulse generator incorporating frequency and duty

- 14 -

cycle modulation, which modulation is realized by means of changes in the input voltage which as will be explained is provided by a feedback path from the flow cell 10.

The pulse output 32 of the pulse generator 30 is applied via resistor R3 to the flow cell 10. The current flow to the flow cell 10 is controlled by resistors R3, R4 and R5. At specific flow rates, the current passing through the cell 10 can be adjusted by means of adjusting the values of resistors R3 and/or R5. The output of the flow cell taken across resistor R5 is applied to an RC network comprises of resistor R6 and capacitor C2 which integrates the pulses applied from the flow cell 10 to a dc value. This dc output, which is proportional to the current through the cell, is applied to operational amplifier A1, the gain of which is controlled by resistors R7 and R8. The output of the operational amplifier A1 is applied to an RC circuit comprises of resistor R9 and capacitor C2 which filters the output.

The filtered output from the operational amplifier A1 is applied in a feedback path through a buffer amplifier B1 to the input of the pulse generator 30. As states before, the feedback voltage controls the output duty cycle and/or frequency of the dc pulse generator 30.

- 15 -

In addition, the filtered output from amplifier A1 is applied via an adding circuit comprises of amplifiers A2 and A3 which serve simply to shift the modulation voltage value onto a range which is suitable for the particular chart recorder which is being used for the measurement. The additional amplification provided by A2 and A3 does not affect the flow rate measurements and is merely utilized for simplifying data collection and the use of specific output recording devices.

The important feature in operation of the circuit described in FIGS. 3 and 4 resides in the stabilization of the impedance between the electrodes 18 and 20. This is achieved by utilizing monopolar current pulses to drive the flow cell 10 which are then integrated to a dc value and fed back to the input of the pulse generator to modulate the pulses which are applied to the flow cell. This procedure quickly stabilizes the impedance between the electrodes permitting an accurate, quick measurement to be made even for very slow flow rates. The performance of the present system can be categorized with respect to response time and the change in output voltage observed as a result of a given change in flow rate. The circuit's response time is defined as the time necessary for the impedance of the flow cell 10 to achieve the specific steady state value dictated by the flow rate present. Optimization of circuit performance involves minimizing the response time while maximizing the observed changes in output voltage which are induced by given changes in flow rates. It has been found that each of the above performances is mediated by the current density through the flow cell. At a specific flow rate as indicated above, the current passing through the cell 10 can be adjusted in accordance with the present circuit by changing the values of R3 and R5. As also been indicated, current density can be adjusted by varying the electrode geometry.

Dimensions have been provided on FIGS. 1 and 2 for the flow cell which is illustrative of a preferred form of the flow cell which may be utilized in the present invention. This specific flow cell may be utilized for an implant in the human body for measuring cerebrospinal bulk flow rates since it is the same size as the shunt tubing and can be implanted therewith. It will be appreciated that different types of cells may be utilized for different applications. Furthermore, in accordance with the present invention, the methods and apparatus provided herein may include the suspension of shaped parallel electrodes in different types of flow cells which maintain contact with the fluid being measured while minimizing disturbances with the flow rates. This is particularly required when the rates of flow are extremely slow. The magnitude of the circuit components illustrated in FIG. 4 simply constitute an illustrative embodiment for a particular application and may be varied to fit various operating requirements and environments.

If the magnitudes of the flow rates and the corresponding output voltages they induce are statistically analyzed, a near perfect logarithmic relationship results with a coefficient of determination ($R^2$) of 0.99. In accordance with the present invention, flow voltage data has been obtained covering well over two orders of magnitude for a given set of circuit component values. These limitations represent circuit limitations and not breakdowns of/or departures from the aforementioned phenomena and relationships. Specifically, flow voltage data has been obtained from a low value of .074 ml/min through 14 successively increasing flow values to a maximum of 24.7ml/min. These values have also been repeatably reproducible.

It is also believed that the operation of the flow cell can be improved by coating the surface of the electrodes which contact the cerebrospinal fluid with teflon. The teflon coating is porous to ions and thus would not hamper the passage of current, while at the same time the coating would block proteins in the cerebrospinal fluid. It is believed that the proteins contribute to a major portion of the error in the flow measurements.

Since other changes and modifications varied to fit particular operating requirements and environments will be apparent to those skilled in the art, the invention is not considered limited to the examples chosen for purposes of illustration, and covers all changes and modifications which do not constitute a departure from the true spirit and scope of this invention.

Claims :

1. A device for measuring the flow rate of electrolytic fluids, comprising:

a flow cell including means forming a cylindrical flow channel receptive of the fluid flow to be measured and a pair of spaced apart electrodes configured to conform to the cylindrical surface of the flow channel and to be flush therewith, the electrodes comprising electrically conductive material inert to the fluid of the flow to be measured, wherein the channel is configured to effect contact of the electrodes with the fluid flow for a desired range of flow rate measurement;

means for applying a monopolar pulse train across the flow cell electrodes having a selected amplitude, period and pulse width to effect a cell impedance across the electrodes which is inversely proportional to the the rate of flow, the pulse applying means including means for varying the current applied across the electrodes in a given unit of time in response to a control signal applied thereto; and

means for producing the control signal in response to the potential across the electrodes in the given unit of time and for applying the control signal to the means for varying the pulse train current to effect an increase in current in response to an increase in the flow rate, whereby the cell impedance is stabilized for every flow rate and the electrode potential is representative of the flow rate.

2. The device according to claim 1, for the measurement of cerebrospinal fluid shunt flow, wherein the means forming cylindrical flow channel includes inlet means ·outlet means for connecting same in series with a cerebrospinal fluid shunt tube, a cylindrical outer surface configured to be substantially equal in diameter to that of the shunt tube and the inner diameter of the fluid channel is substantially equal in diameter to that of the shunt tube, whereby the flow cell is surgically implantable with the shunt.

3. The device according to claim 1 or 2, wherein pulse train applying means comprises a variable pulse generator and the means for producing the control signal comprises an integrator and a variable gain amplifier.

4. A method of measuring the flow rate of electrolytic fluids, comprising the steps of:

a. a connecting flow cell having a cylindrical flow channel in series with the fluid flow;

b. providing a pair of spaced apart electrodes configured to conform to the cylindrical surface of the flow channel and flush therewith and comprising an electrically conductive material inert to the fluid of the flow to be measured to effect contact of the electrodes with the fluid flow for a desired range of flow rate measurement;

c.   applying a monopolar pulse train across the flow cell electrodes having a selected amplitude, period and pulse width to effect a cell impedance across the electrodes which is inversely proportional to the rate of flow;

d.   producing a control signal in response to the potential across the electrodes in a given unit of time;

e.   varying the pulse train current to effect an increase in current in response to an increase in the flow rate whereby the cell impedance is stabilized for every flow rate and the electrode potential is representative of the flow rate.

5.   The method according to claim 4, for the measurement of cerebrospinal fluid shunt flow, comprising connecting shunt tubing to a patient to establish a cerebrospinal fluid shunt, forming the flow channel with a cylindrical outer surface configured to be substantially equal in diameter to that of the shunt tubing and with the inner diameter of the fluid channel substantially equal in diameter to the inner diameter of the shunt tubing, and connecting the flow cell in series with the shunt tubing.

6.   The method according to claim 5, further comprising implanting the flow cell in the patient with the shunt tubing.

7. A surgically implantable flow cell for effecting the measurement of cerebrospinal fluid shunt flow, the cell comprising:

means forming cylindrical flow channel having inlet means outlet means for connecting same in series with an cerebrospinal fluid shunt; and

a pair of spaced apart electrodes configured to conform to the cylindrical surface of the flow channel and flush therewith, the electrodes comprising electrically conductive material which is inert to cerebrospinal fluid.

8. The cell according to claim 7, wherein for a given shunt tube, the means forming the flow channel has a cylindrical outer surface configured to be substantially equal in diameter to that of the shunt tube and the inner diameter of the fluid channel is substantially equal in diameter to that of the shunt tube.

9. Apparatus for measuring flow rates in a fluid which conducts current comprising:

an electrolytic flow cell having said fluid to be measured passing therethrough,

said flow cell having a pair of spaced electrodes in contact with said fluid passing through said flow cell,

pulse generator means for controllably providing monopolar pulses having a selectable amount of current per unit time,

means for coupling said monopolar pulses to said electrodes of said flow cell,

means for deriving a direct current output from said cell proportional to the current flow through said electrodes,

feedback means for coupling said direct current output to said pulse generator means for controlling the current of said monopolar pulses per unit time to thereby stabilize the direct current output from said electrodes to produce a different value for each flow rate whereby a flow rate measurement can be made.

10. The apparatus set forth in claim 9, in which said flow cell comprises an enclosed body having a channel therethrough through which said fluid passes.

11. The apparatus set forth in claim 10, in which said electrodes are shaped to conform to the shape of said channel and are recessed therein.

12. The apparatus set forth in claim 10, in which said pulse generator means has a variable frequency and duty cycle which is controlled by said feedback means.

13. A method of measuring slow flow rates of an electrolytic fluid moving past a pair of spaced electrodes comprising the steps of:

applying a monopolar pulse train across said electrodes having a selected amount of current per unit time,

detecting and amplifying the output from said spaced electrodes, and

feeding back the amplified output from said spaced electrodes for controlling the current per unit time of said pulse train across said electrodes for stabilizing the output from said electrodes.

14. The method set forth in claim 13, including the steps of:

incorporating the electrodes in an enclosed flow cell with an opening therethrough, and

implanting said flow cell in the body of a patient for determining the flow rate of a body fluid.

-.-.-.-.-.-

1/1

0077413

FIG. 1.

FIG. 2.

ELECTRODES

FIG. 3.

VAR. PULSE GEN.

INT.

AMP.

VAR. GAIN

FIG. 4.

555 TIMER

FEEDBACK

FLOW CELL

TO CHART RECORDER

European Patent Office

**PARTIAL EUROPEAN SEARCH REPORT**
which under Rule 45 of the European Patent Convention shall be considered, for the purposes of subsequent proceedings, as the European search report

Application number

**0077413**

EP 81108571.1

## DOCUMENTS CONSIDERED TO BE RELEVANT

| Category | Citation of document with indication, where appropriate, of relevant passages | Relevant to claim |
|---|---|---|
| D,A | US - A - 3 450 984 (HOLMES)<br><br>* Abstract; column 2, lines 48-64; fig. 3 * | 1,4,9, 13 |
| A | US - A - 3 347 224 (ADAMS)<br><br>* Claim 1; fig. 1,2 * | |
| A | GB - A - 1 392 926 (GEORGE KENT LTD)<br><br>* Claim 1; fig. 1 * | |
| D,A | US - A - 3 712 134 (DETTMER)<br><br>* Totality * | |
| A | IEEE TRANSACTIONS ON BIOMEDICAL ENGINEERING, vol. BME-26, no. 12, December 1979, New York<br><br>KATSUYUKI SAKAMOTO AND HIROSHI KANAI "Electrical Characteristics of Flowing Blood"<br><br>* Pages 686,687; fig. 3 * | 1,4,9, 10 |

**CLASSIFICATION OF THE APPLICATION (Int Cl³)**

G 01 F 1/56

A 61 B 5/00

**TECHNICAL FIELDS SEARCHED (Int Cl³)**

G 01 F 1/00

A 61 B 5/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-4, 7-13
Claims searched incompletely: –
Claims not searched: 5,6,14
Reason for the limitation of the search:

Article 52(4)

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family,

corresponding document

| Place of search | Date of completion of the search | Examiner |
|---|---|---|
| VIENNA | 23-06-1982 | STÖGER |